# EUROPÄISCHE PATENTSCHRIFT

(11) **EP 3 057 543 B1**
(45) Veröffentlichungstag und Bekanntmachung des Hinweises auf die Patenterteilung: **23.08.2017**
(21) Anmeldenummer: 14789522.1
(22) Anmeldetag: 14.10.2014
(51) Int. Cl.: A61F 5/01, A61F 5/058

(54) **DAUMENORTHESE**
THUMB ORTHOSIS
ORTHÈSE DE POUCE

(30) Priorität: 16.10.2013 DE 102013017167
(43) Veröffentlichungstag der Anmeldung: 24.08.2016
(73) Patentinhaber: Otto Bock HealthCare GmbH, 37115 Duderstadt (DE)
(72) Erfinder: ANDERSSON, Ulf, S-582 34 Linköping (SE); NYLIN, Britt, S-614 34 Söderköping (SE); BOHLIN, Johan, 614 30 Söderköping (SE); OLSSON, Frederik, 64135 Katrineholm (SE)
(74) Vertreter: Friedrich, Andreas
(86) Internationale Anmeldenummer: PCT/EP2014/002770
(87) Internationale Veröffentlichungsnummer: WO 2015/055304

(56) Entgegenhaltungen:
- US-A- 2 369 210
- US-A- 5 928 172
- US-A- 6 101 628
- US-B1- 7 854 714

## Beschreibung

Die Erfindung betrifft eine Daumenorthese mit einer Daumenmanschette zum Umfassen eines Daumens und wenigstens einem daran befestigten Befestigungsband.

Daumenorthesen sind in vielfältiger Form aus dem Stand der Technik bekannt. Sie werden verwendet, um beispielsweise nach einer Verletzung des Daumens bzw. eines der Daumengelenke das entsprechende Gelenk zu schonen und vor Überanstrengung und erneuter Verletzung zu schützen. Auch als präventive Maßnahme gegen eine Überbeanspruchung des Daumens bzw. eines seiner Gelenke ist die Verwendung einer Daumenorthese bekannt.

Eine herkömmliche Daumenorthese umfasst dabei zumeist eine Daumenmanschette, die den Daumen mehr oder weniger weit umfasst. Es sind Manschetten aus einem flexiblen Material bekannt, die den Daumen nahezu vollständig umgeben, während andere Daumenmanschetten beispielsweise nur das Daumengrundgelenk umfassen. Auch die Verwendung von unelastischen Materialien, beispielsweise steifen Kunststoffen, ist bekannt. Eine derartige Daumenmanschette macht die Bewegung des Daumens nach dem Anlegen der Daumenorthese nahezu unmöglich und führt somit zu einer fast vollständigen Stilllegung in einer Schonhaltung. An der Daumenmanschette ist zumeist wenigstens ein Befestigungsband befestigt, das beispielsweise um das Handgelenk des Trägers der Daumenorthese gelegt wird und so die Daumenmanschette in der gewünschten Position fixiert. Auch hier sind unterschiedlichste Ausführungsformen aus dem Stand der Technik bekannt. So gibt es Daumenorthesen, die neben dem Daumengrundgelenk und ggf. eines Großteil des Daumens auch noch das nahezu vollständige Handgelenk des Trägers der Orthese umfassen, um auch dieses Gelenk ruhig zu stellen und zu schonen. Andere Daumenorthesen bestehen neben der Daumenmanschette lediglich aus einem relativ schmalen Befestigungsband, das beispielsweise einmal um den Handrücken oder das Handgelenk des Trägers gelegt wird um danach an der Daumenmanschette beispielsweise über ein Klettverschlusselement befestigt wird.

Nachteilig ist jedoch bei diesen Daumenorthesen, das zum einen in der Regel nicht einstellbar ist, wie stark der Daumen bzw. das zu schonende Gelenk ruhiggestellt und in welchem Umfang eine weitere Bewegung noch ermöglicht werden soll. Soll beispielsweise im Laufe einer Rehabilitation nach einer Verletzung des Daumens die Bewegungsfähigkeit schrittweise erhöht werden, mussten herkömmlicherweise unterschiedliche Daumenorthese für den Patienten bereitgestellt werden. Dies ist kostenintensiv und zudem für den Patienten unbequem, da er nicht selbst den Grad der Einschränkung der Bewegung einstellen kann.

Zum anderen sind Daumenorthesen aus dem Stand der Technik oftmals so ausgebildet, dass eine am Daumen bzw. der Hand des Trägers angelegte Daumenorthese einen Großteil der Handinnenfläche des Trägers der Daumenorthese abdeckt. Insbesondere bei der Verwendung von starren und unflexiblen Kunststoffen führt dies dazu, dass nicht nur der Daumen in seiner Bewegungsfähigkeit wie gewollt eingeschränkt ist, sondern dass zusätzlich die gesamte Hand in ihrer Funktionsfähigkeit eingeschränkt ist. Ein sicheres Greifen ist mit einer derartigen Daumenorthese nicht oder nur in sehr eingeschränktem Maße möglich.

Aus der US 7,854,714 B1 ist eine Daumenorthese bekannt, die eine Daumenmanschette zum Umfassen des Daumens und ein daran befestigtes Befestigungsband aufweist. Zusätzlich verfügt sie über eine Schlaufe, die an dem Befestigungsband angeordnet ist und aus einem elastischen Material besteht. An ihr kann der Träger der Orthese beim Anlegen der Daumenorthese ziehen, um das Befestigungsband und insbesondere die Daumenmanschette elastisch vorzuspannen.

Aus der US 6,101,628 ist eine Handorthese bekannt, die Vibrationen und Schläge dämpfen und abfedern soll.

Der Erfindung liegt folglich die Aufgabe zugrunde, eine gattungsgemäße Daumenorthese so weiterzuentwickeln, dass der Grad der dem Daumen verbleibenden Bewegungsfreiheit nahezu stufenlos einstellbar ist und trotzdem ein möglichst großer Anteil der Handinnenfläche nicht von der Orthese abgedeckt wird, sodass die Hand zumindest nahezu vollständig funktionsfähig und verwendbar bleibt.

Die Erfindung löst die gestellte Aufgabe durch eine Daumenorthese gemäß dem Oberbegriff des Anspruchs 1, die sich dadurch auszeichnet, dass das Befestigungsband ein Loch zum Hindurchstecken des Daumens aufweist. Durch die konstruktiv einfache Ausgestaltung des Befestigungsbandes mit einem Loch ist es möglich, die Daumenorthese in besonders einfacher und dennoch sicherer Weise am Daumen bzw. der Hand des Trägers der Daumenorthese zu befestigen und gleichzeitig einen möglichst kleinen Teil der Handinnenfläche abzudecken. Wird eine derartige Daumenorthese angelegt, wird zunächst der Daumen in die Daumenmanschette so eingeführt, dass die Daumenmanschette den Daumen umfasst. Dies ist noch analog zu Daumenorthesen aus dem Stand der Technik. Anschließend wird das Befestigungsband um das Handgelenk des Trägers der Daumenorthese herumgeführt. Anders als bei Orthesen aus dem Stand der Technik wird jedoch nun nicht das Befestigungsband an der Daumenmanschette befestigt, sondern der Daumen wird durch das Loch im Befestigungsband hindurchgesteckt. Anschließend wird der verbleibende Rest des Befestigungsbandes beispielsweise an der Daumenmanschette befestigt.

Bei Daumenorthesen aus dem Stand der Technik wird die auf den Daumen wirkende Kraft durch das Befestigungsband ausgeübt, das zugbelastet im angelegten Zustand der Daumenorthese an der Daumenmanschette befestigt ist. Herkömmlicherweise wird das Befestigungsband außen, also über den Handrücken an der Hand des Trägers entlang geführt, sodass auf den Daumen nur eine schwer kontrollierbare und nahezu nicht einstellbare Kraft nach außen (dorsal) in Richtung auf den Handrücken wirkt. In Abhängigkeit davon, wie groß die Zugkraft ist, die beim Anlegen der Daumenorthese aus dem Stand der Technik auf das Befestigungsband ausgeübt wird, ändert sich auch die auf den Daumen aufgebrachte Kraft. Eine reproduzierbare Einstellung insbesondere vom Träger der Daumenorthese selbst ist daher nur schwer oder sogar gar nicht möglich. Bei einer erfindungsgemäßen Daumenorthese ist die aufgebrachte Zugkraft durch die Position des Lochs im Befestigungsband festgelegt. Das Befestigungsband kann auf diese Weise beim Anlegen beispielsweise in einem genau festgelegten Maße gedehnt werden, wobei die Dehnung dadurch festgelegt wird, dass einerseits der Daumen in der Daumenmanschette eingeführt ist und andererseits der Daumen durch das Loch im Befestigungsband geführt werden muss. Dies hat zum einen zur Folge, dass nicht nur eine nach außen in Richtung auf den Handrücken wirkende Kraft auf das Daumengelenk wirkt, sondern auch eine entgegengesetzte Kraft, sodass das Daumengrundgelenk beispielsweise in beide Richtungen zumindest nahezu gleich stark in der Bewegung eingeschränkt wird und führt zum anderen zu einer Reproduzierbarkeit dieser Kräfte und damit auch zu einer Reproduzierbarkeit der Bewegungseinschränkung des Daumens. Dies ist insbesondere bei Rehabilitationsmaßnahmen wünschenswert.

Das Loch im Befestigungsband befindet sich vorteilhafterweise nicht an einem Ende, sondern beispielsweise im mittleren oder sogar in einem der Daumenmanschette zugewandten Anteil des Befestigungsbandes. Dies bedeutet, dass nach dem Herumführen des Befestigungsbandes um die Hand bzw. das Handgelenk des Trägers der Daumenorthese und das anschließende Hindurch-stecken des Daumens durch das Loch noch ein gewisser Anteil des Befestigungsbandes übrig ist, der erneut um das Handgelenk oder den Handrücken oder in sonstiger Weise um die Hand herumgeführt werden kann. Da dies auf unterschiedlichen Wegen geschehen kann, ist auf diese Weise der Grad der Bewegungseinschränkung für den Daumen nahezu stufenlos und frei einstellbar. Auch dies hat insbesondere für Rehabilitationszwecke eine vorteilhafte Wirkung, da beispielsweise im Laufe der Behandlung die Bewegungseinschränkung reduziert und so der Daumen wieder an seine volle Belastung gewöhnt werden kann.

Vorteilhafterweise weist die Daumenmanschette wenigstens eine Schiene zum Stabilisieren wenigstens eines Teils des Daumens auf. Diese Schiene kann beispielsweise in das Material der Daumenmanschette eingenäht oder auch auf der Außen- oder Innenseite der Daumenmanschette aufgenäht oder aufgeklebt sein. Auf diese Weise ist es möglich, unterschiedliche Gelenke innerhalb des Daumens unterschiedlich stark zu fixieren und so beispielsweise das Daumengrundgelenk nur in der Bewegung einzuschränken, während beispielsweise die weiteren Fingerglieder des Daumens in einer bestimmten Richtung an einer Bewegung gehindert werden. Vorteilhafterweise ist die Schiene dabei an die gewünschte Form des Daumens angepasst. Insbesondere kann die Schiene zumindest einen Teil des Daumens teilweise umfassen, sodass eine optimale Passform gewährleistet werden kann. Über die Länge und den Teilbereich des Daumens, der von der Schiene umfasst wird, lässt sich der Grad der Bewegungseinschränkung und damit der Ruhigstellung des Daumens einstellen.

Vorteilhafterweise besteht die Schiene aus einem an die Form des Daumens anformbaren Material. Vorteilhafterweise ist das anformbare Material ein Kunststoff oder ein Metall. Als Schiene kann beispielsweise ein Metallstreifen geringer Dicke gewählt werden, der zwar eine ausreichende Festigkeit und Formstabilität besitzt, um den Daumen in einer gewünschten Position zu halten oder zumindest die Bewegungsfreiheit des Daumens einzuschränken, der jedoch eine so geringe Dicke aufweist, dass beim Anlegen und Anformen der Orthese an den Daumen eine Veränderung der Gestalt dieser Schiene möglich ist. Dies hat den Vorteil, dass eine Individualisierung der Schiene und damit der Daumenorthese für jeden einzelnen Patienten bzw. Träger der Daumenorthese beim Anlegen der Orthese erfolgt, sodass nicht eine Vielzahl unterschiedlicher Formen für unterschiedliche Personen vorgehalten werden muss oder für den Träger einer Daumenorthese ein individuelles Exemplar angefertigt werden muss.

Vorteilhafterweise ist die Schiene an einer Innenseite der Daumenmanschette angeordnet. Als besonders vorteilhaft hat sich herausgestellt, wenn die Schiene in die Daumenmanschette, insbesondere in eine an der Daumenmanschette angeordnete Tasche, herausnehmbar eingeführt ist. Dies bedeutet insbesondere, dass die Schiene ein separates Bauteil ist, sodass die Schiene beispielsweise an dem Daumen angeordnet und an die Form des Daumens angeformt werden kann, bevor der Daumen in die Daumenmanschette eingeführt wird. Dies ist insbesondere beim erstmaligen Verwenden der Daumenorthese von Vorteil, da es ein besonders einfachen und bequemes Anformen der Schiene an die gewünschte Form des Daumens ermöglicht. Ist die Schiene dann in der gewünschten Form kann die Daumenmanschette auf zwei unterschiedliche Weisen angelegt werden, wenn die Schienen aus der Daumenmanschette herausnehmbar ausgebildet ist. Entweder kann zunächst die Schiene an dem Daumen angeordnet werden, woraufhin dann der Daumen mit der Schiene in die Daumenmanschette eingeführt wird. Alternativ dazu lässt sich auch zunächst die Schiene in die Daumenmanschette einführen bevor der Daumen in die Daumenmanschette eingeführt wird. Beide Möglichkeiten sind denkbar und können dem jeweiligen Empfinden und Gefühl des Trägers der Daumenorthese entsprechend ausgewählt werden.

Anstatt die Schiene auf die eine oder andere Art direkt an der Haut des Trägers der Daumenorthese anzuordnen, kann sie auch in einer bevorzugten Ausgestaltung der hier beschriebenen Daumenorthese in einer Tasche der Daumenmanschette angeordnet werden. Dadurch wird ein direkter Hautkontakt der Schiene vermieden während gleichzeitig die Vorteile bestehen bleiben, die sich daraus ergeben, dass die Schiene als separates Bauteil ausgebildet ist. Die Tasche, in die die Schiene einschiebbar ist, kann dabei beispielsweise durch ein Klettverschlusselement verschließbar ausgebildet sein. Oftmals ist es jedoch auch ausreichend, eine nicht verschließbare Tasche zu verwenden.

Vorteilhafterweise besteht das Befestigungsband zumindest abschnittsweise aus einem elastischen Material. Auf diese Weise ist gewährleistet, dass eine Dehnung des Befestigungsbandes beim Anlegen der Daumenorthese hervorgerufen werden kann, sodass eine Zugbelastung entsteht, die in gewünschter Weise die Kräfte auf das Daumengelenk bzw. den Daumen des Trägers der Daumenorthese ausübt. Vorteilhafterweise ist jedoch das Loch in dem Befestigungsband von einem unelastischen Material umgeben. Insbesondere an dieser Stelle sind oftmals keine Dehnungen des Befestigungsbandes erwünscht, da es oftmals zu einem direkten Hautkontakt kommt, sodass Dehnungen im Befestigungsband zu Wunden und Druckstellen führen können.

Vorzugsweise weist das Befestigungsband ein der Daumenmanschette abgewandtes Ende auf, das an der Daumenmanschette befestigbar ist. Dadurch wird die Daumenorthese beispielsweise am Handgelenk oder der Hand des Trägers der Daumenorthese befestigt und kann so bei der Benutzung der Hand nicht verloren werden. Dabei hat es sich als vorteilhaft herausgestellt, wenn sowohl an dem Ende des Befestigungsbandes als auch an der Daumenmanschette Klettverschlusselemente angeordnet sind, durch die die einzelnen Elemente aneinander befestigbar sind. Vorteilhafterweise verfügt die Daumenmanschette über einen Großteil ihrer Außenfläche insbesondere in einem dem Handgelenk zugewandten Anteil über eines der Klettverschlusselemente, sodass das Ende des Befestigungsbandes in unterschiedlichen Positionen vorteilhafterweise stufenlos verstellbar an der Manschette angeordnet werden kann, sodass die aufgebrachte Kraft stufenlos je nach Bedarf eingestellt werden kann.

Durch die hier beschriebene Daumenorthese lassen sich unterschiedliche Effekte der Daumenorthese erreichen, ohne dass hierfür verschiedene Ausgestaltungen einer Daumenorthese vorgehalten werden müssen. Wird beispielsweise das Befestigungsband nach dem Hindurchstecken des Daumens durch das Loch im Befestigungsband um das Handgelenk des Trägers der Daumenorthese herumgewickelt und anschließend beispielsweise das Ende des Befestigungsbandes an der Daumenmanschette angeordnet, bietet die Daumen-orthese einen Schutz gegenüber der Dehnung des Daumens. Wird hingegen das Befestigungsband beispielsweise um den Daumen herumgewickelt oder beispielsweise zwischen dem Daumen und den übrigen Fingern der Hand hindurchgeführt, bietet die Daumenorthese einen Schutz gegen eine Beugung des Daumens und damit Schutz gegen eine Überbeanspruchung in die genau entgegengesetzte Richtung. In beiden Ausführungsformen bleibt jedoch ein Großteil der Handinnenfläche von der Daumenorthese unüberdeckt, sodass die Hand weiterhin nahezu vollständig einsatzfähig bleibt.

Vorzugsweise ist insbesondere die Innenseite der Daumenmanschette zumindest teilweise beschichtet. Dies kann beispielsweise eine TPE- oder Silikonbeschichtung sein, die dafür sorgt, dass eine gute Haftung zwischen der Daumenorthese und der Haut des Trägers erreicht wird. Dies ist insbesondere dann von Vorteil, wenn die Orthese beispielsweise bei Rehabilitationsmaßnahmen von Sportverletzungen verwendet wird, da in diesem Fall mit der angelegten Daumenorthese Sport getrieben wird. Dabei kann es zu großen mechanischen Belastungen kommen, die zu einem Verrutschen der Daumenorthese an der Hand des Trägers führen können. Dadurch wird nicht nur der Tragekomfort der Daumenorthese deutlich verringert, sondern auch die Wirkung der Daumenorthese als Schutz für ein bereits geschwächtes oder verletztes Gelenk verringert oder sogar vollständig verhindert.

Zusätzlich kann alternativ dazu auch die Außenseite der Daumenmanschette zumindest teilweise beschichtet sein. Dies kann mit den gleichen Materialien geschehen, mit denen auch die Innenseite der Daumenmanschette zumindest teilweise beschichtet ist. Natürlich ist auch eine vollständige Beschichtung der Innenseite und/oder der Außenseite der Daumenmanschette möglich. Eine Beschichtung auf der Außenseite der Daumenmanschette ist insbesondere dann von Vorteil, wenn mit der Manschette Sport getrieben werden soll, bei dem es sich insbesondere um Ballsportarten, beispielsweise Handball oder Volleyball, handelt. In diesem Fall kann eine flächige oder teilweise Beschichtung, insbesondere im Bereich des Daumens und/oder des Handtellers der Daumenmanschette oder auch anderer Teile der Daumenorthese von Vorteil sein.

Als vorteilhaft hat sich auch herausgestellt, die Schiene, die zum Stabilisieren wenigstens eines Teiles des Daumens vorgesehen, ist gepolstert und/oder beschichtet ist. Die Polsterung kann über einen Schaumstoff oder ein sonstiges Polstermaterial, wie es aus dem Stand der Technik per se bekannt ist, geschehen. Auf diese Weise wird einerseits die Stützwirkung der Schiene nicht beeinträchtigt und andererseits der Tragekomfort der Daumenorthese erhöht. Die Schiene befindet sich zumeist an der Außenseite des Daumens und daher an einer durchaus exponierten und empfindlichen Stelle, sodass insbesondere bei großer mechanischer Belastung es durch eine ungepolsterte Schiene zu Druckstellen und schmerzhaften Blessuren kommen könnte. Durch Polsterung der Schiene wird dieser Gefahr wirksam begegnet.

Mit Hilfe einer Zeichnung wird nachfolgend ein Ausführungsbeispiel der vorliegenden Erfindung näher erläutert. Es zeigt:
- Figur 1: die schematische Darstellung einer Daumenorthese gemäß einem ersten Ausführungsbeispiel der vorliegenden Erfindung;
- Figur 2: die Darstellung aus Figur 1 mit Schiene;
- Figur 3a, 3b: die schematische Darstellung einer Hand mit unterschiedlich ausgestalteten Schienen für eine Daumenorthese gemäß einem weiteren Ausführungsbeispiel der vorliegenden Erfindung;
- Figur 4: die schematische Darstellung einer Daumenorthese, bei der der Daumen in die Daumenmanschette eingeführt wurde,
- Figuren 5a-5g: verschiedene Stadien beim Anlegen einer Daumenorthese gemäß einem Ausführungsbeispiel der vorliegenden Erfindung und
- Figur 6 -: die schematische Darstellung einer weiteren Daumenorthese gemäß einem Ausführungsbeispiel der vorliegenden Erfindung.

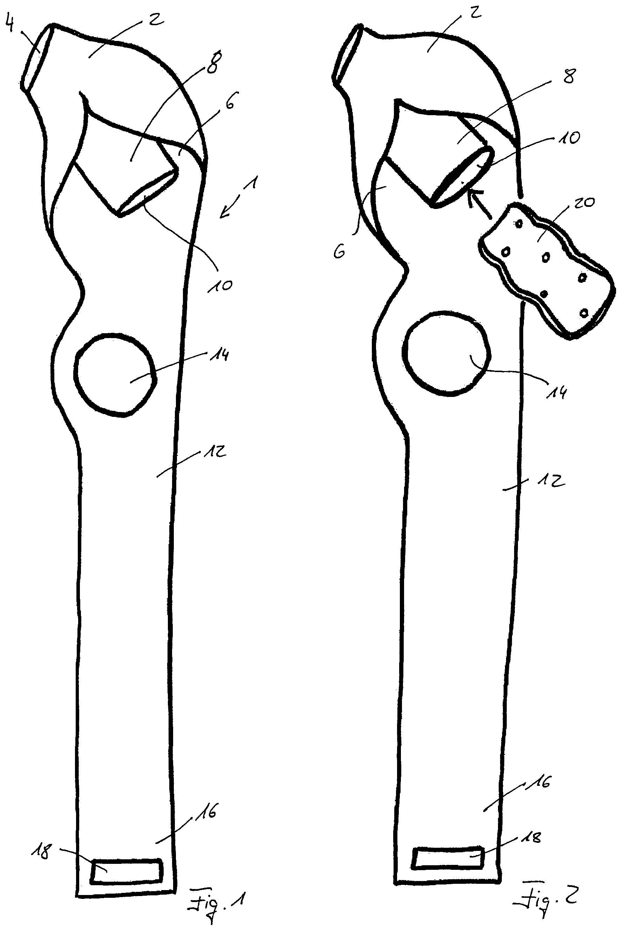

Figur 1 zeigt eine Daumenorthese 1 gemäß einem ersten Ausführungsbeispiel der vorliegenden Erfindung. Sie verfügt an ihrem oberen Ende über eine Daumenmanschette 2, die eine Öffnung 4 aufweist, durch die ein Daumen des Trägers der Daumenorthese 1 hindurchgeführt wird. An einer Innenseite 6 der Daumenorthese befindet sich eine Tasche 8 mit einer Taschenöffnung 10, durch die eine in Figur 1 nicht dargestellte Schiene einführbar ist.

An die Daumenmanschette 2 schließt sich ein Befestigungsband 12 an, das über ein Loch 14 verfügt. An einem der Daumenmanschette abgewandten Ende 16 des Befestigungsbandes 12 befindet sich ein Klettverschlusselement 18, durch das das Ende 16 des Befestigungsbandes 12 beispielsweise an der Daumenmanschette 2 befestigbar ist.

Figur 2 zeigt die Darstellung aus Figur 1 mit einer Schiene 20, die entlang des dargestellten Beispiels durch die Taschenöffnung 10 in die Tasche 8 an der Innenseite 6 der Daumenmanschette 2 einführbar ist.

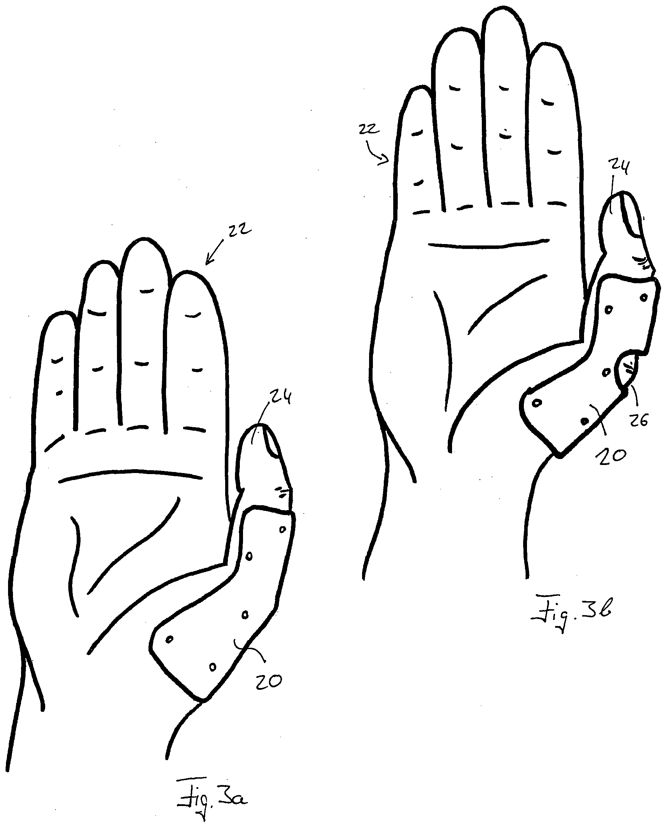

Die Figuren 3a und 3b zeigen die schematische Darstellung jeweils einer Hand 22 wobei sich an einem Daumen 24 dieser Hand 22 jeweils eine Schiene 20 befindet. Beide Schienen 20 in den Figuren 3a und 3b sind an die gewünschte Form des Daumens 24 angeformt. Der Unterschied zwischen den beiden Schienen 20 besteht lediglich darin, dass die Schiene 20 in Figur 3b über eine Ausnehmung 26 verfügt die für ein Gelenk des Daumens 24 vorgesehen ist. Nachdem die Schienen 20 so wie in den Figuren 3a und 3b gezeigt am jeweiligen Daumen 24 angeordnet sind, kann der Daumen 24 durch die Daumenmanschette 2 geführt werden, sodass die Schiene 20 am Daumen 24 festgelegt wird.

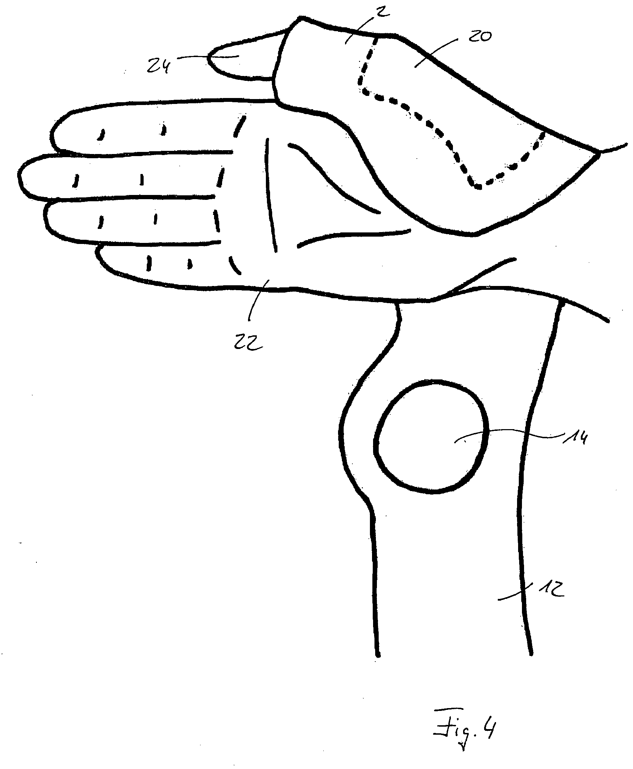

Figur 4 zeigt diese Situation. Der Daumen 24 ist durch die Daumenmanschette 2 hindurchgeführt, sodass sich die Schiene 20, die in Figur 4 nur durch gestrichelte Linien dargestellt ist, nun zwischen der Daumenmanschette 2 und dem Daumen 24 befindet. Dadurch wird sie in der gewünschten Position an der Hand 22 bzw. dem Daumen 24 festgelegt.

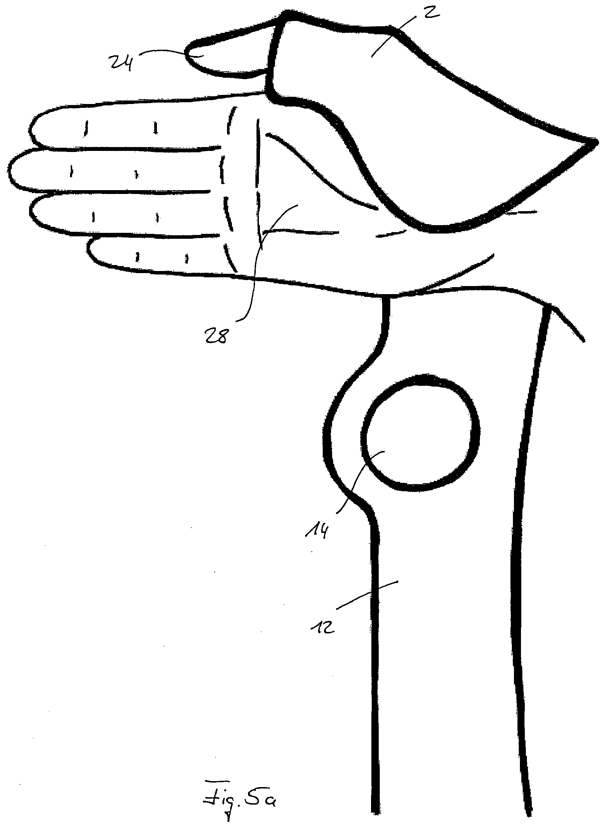

In den Figuren 5a bis 5g werden unterschiedliche Stadien des Anlegens einer Daumenorthese 1 gemäß einem Ausführungsbeispiel der vorliegenden Erfindung näher erläutert. Zunächst wird, wie in Figur 5a dargestellt, der Daumen 24 durch die Daumenmanschette 2 hindurchgeführt. An der Innenseite 6 der Daumenmanschette 2 kann die Schiene 20 angeordnet sein. Alternativ kann sie auch in einer Tasche 8 oder in das Material der Daumenmanschette 2 eingenäht vorhanden sein. In Figur 5a ist die Hand 22 von der Seite ihrer Handinnenfläche 28 dargestellt. Nach diesem in Figur a gezeigten ersten Schritt hängt das Befestigungsband 12 mit dem Loch 14 lose über den Handrücken der Hand 22 herunter.

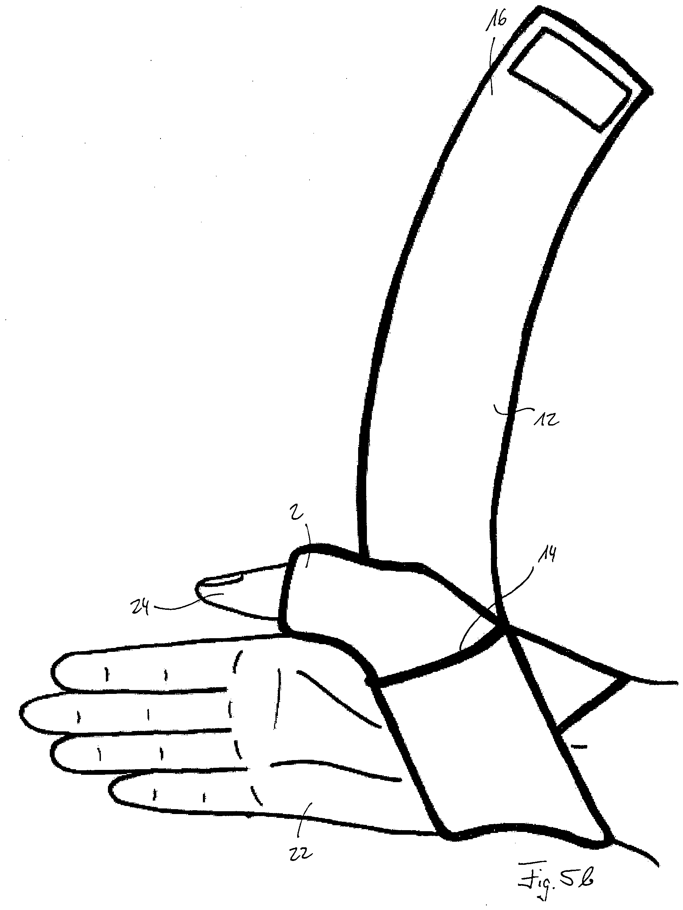

Figur 5b zeigt den nächsten Schritt. Das Befestigungsband 12 ist um das Handgelenk des Trägers der Daumenorthese 1 herumgelegt worden und der Daumen 24 ist mit der Daumenmanschette 2 durch das Loch 14 im Befestigungsband 12 hindurchgeführt worden. Auf diese Weise ist die Daumenorthese nun in nahezu optimaler Weise an der Hand 22 festgelegt.

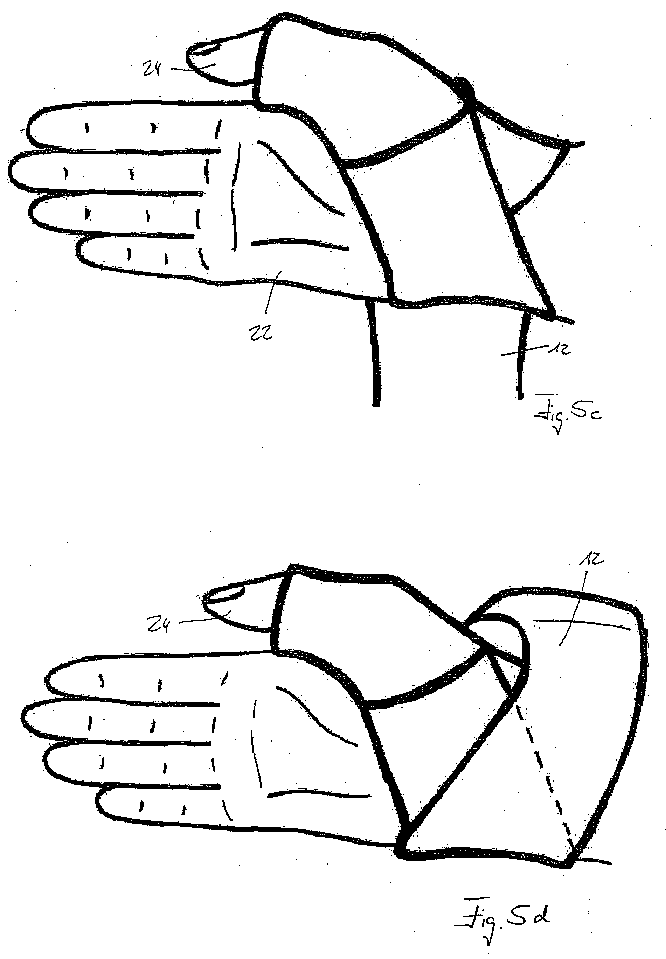

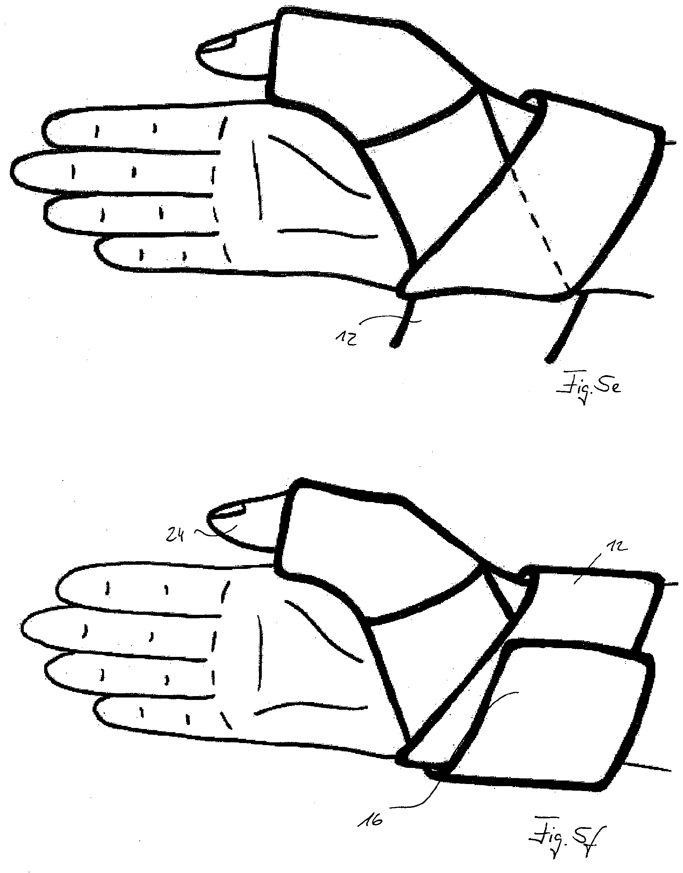

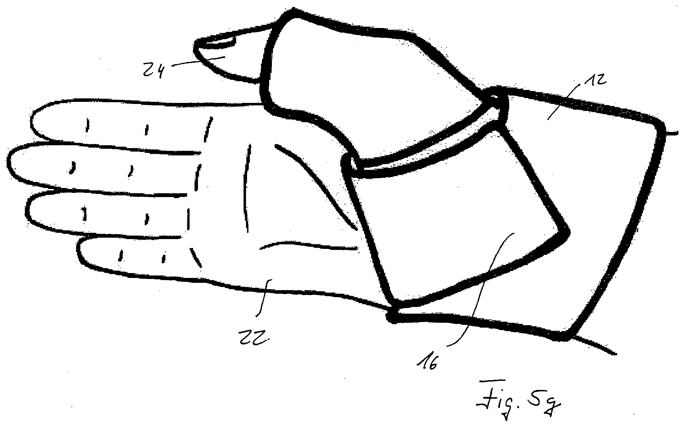

Das in Figur 5b noch nach oben abstehende Ende 16 des Befestigungsbandes 12 ist in Figur 5c bereits nach unten umgeklappt worden und hängt nun auf der Handrückenseite der Hand 22 herunter. Das Befestigungsband 12 kann nun auf unterschiedliche Weise um die Hand 22 bzw. den Daumen 24 oder Handgelenk herumgewickelt und so festgelegt werden. Figur 5d zeigt, dass das Befestigungsband 12 erneut auf der den Fingern abgewandten Seite des Daumens 24 um das Handgelenk des Trägers der Daumenorthese 1 herumgeführt wird. Auf diese Weise wird ein Schutz gegen eine Überdehnung des Daumens erreicht. Figur 5e zeigt das Befestigungsband 12 das nach dem in Figur 5d gezeigten Herumlegen um das Handgelenk wieder auf dem der Handrücken zugewandten Seite der Hand herunterhängt. Das Ende 16 des Befestigungsbandes 12 wird im in Figur 5f gezeigten Beispiel erneut um das Handgelenk herumgewickelt und in diesem Fall nicht an der Daumenmanschette 2 sondern am Befestigungsband 12 selbst festgelegt. Figur 5g zeigt eine alternative Befestigungsart, bei der das Befestigungsband 12, nachdem es in Figur 5e um das Handgelenk herumgelegt wurde, nicht über den Handrücken der Hand geführt sondern zwischen dem Daumen 24 und den übrigen Fingern der Hand 22 hindurchgeführt und erst dann mit dem Ende 16 am Befestigungsband 12 festgelegt wird. Natürlich kann das Befestigungsband 12 festgelegt wird. Natürlich kann das Befestigungsband je nach Länge auch mehr oder weniger häufig um das Handgelenk und/oder um den Daumen der Hand herumgewickelt werden. Auf diese Weise lassen sich unterschiedliche Kräfte auf den Daumen 24 aufbringen, die nicht nur in der Stärke sondern auch in der Richtung der Wirkung der Kraft variieren kann.

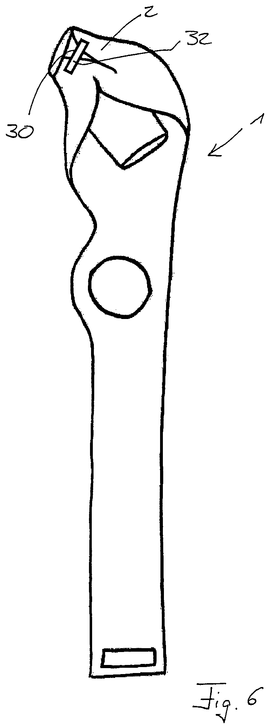

Figur 6 zeigt eine Daumenorthese 1, die der Daumenorthese 1 in Figur 1 entspricht. Der Unterschied besteht darin, dass die Daumenmanschette 2 nicht wie in Figur 1 dargestellt durchgängig geschlossen ist, sondern einen in Figur 6 schematisch dargestellten Schlitz 30 aufweist, der mit einem Befestigungselement 32 überbrückbar ist. Das Befestigungselement 32 ist nur auf einer Seite des Schlitzes 30 unlösbar befestigt, beispielsweise angenäht oder angeklebt. Auf der gegenüberliegenden Seite des Schlitzes befindet sich beispielsweise ein Klettverschlusselement, das mit einem am Befestigungselement 32 angeordneten korrespondierenden Klettverschlusselement so zusammenwirken kann, dass auch das zweite Ende des Befestigungselementes 32 an der Daumenmanschette 2 auf der dem ersten Ende gegenüberliegenden Seite des Schlitzes 30 befestigbar ist. Diese Ausgestaltung hat den Vorteil, dass beim Anlegen der Daumenorthese 1 zunächst das Befestigungselement 32 nur an einer Seite des Schlitzes 30 befestigt ist und der Schlitz 30 dafür sorgt, dass die Daumenmanschette 2 aufgeweitet werden kann. Dies hat zur Folge, dass einerseits die Daumenmanschette 2 leichter angelegt werden und andererseits erreicht wird, dass auf unterschiedliche Daumendurchmesser und Daumendicken reagiert werden kann. Nachdem der Daumen durch die Daumenmanschette 2 eingeführt wurde, wird das Befestigungselement 32 auch auf der der ersten Seite gegenüberliegenden zweiten Seite des Schlitzes 30 befestigt. Insbesondere für den Fall, dass hier eine Klettverbindung vorgesehen ist, kann die Position, in der diese Festlegung geschieht, stufenlos eingestellt werden. Der Träger der Daumenorthese 1 kann sich daher den Umfang der Daumenmanschette 2 individuell auf seine Bedürfnisse einstellen.

### Bezugszeichenliste

- 1: Daumenorthese
- 2: Daumenmanschette
- 4: Öffnung
- 6: Innenseite
- 8: Tasche
- 10: Taschenöffnung
- 12: Befestigungsband
- 14: Loch
- 16: Ende
- 18: Klettverschlusselement
- 20: Schiene
- 22: Hand
- 24: Daumen
- 26: Ausnehmung
- 28: Handinnenfläche
- 30: Schlitz
- 32: Befestigungselement

## Patentansprüche

1. Daumenorthese (1) mit
einer Daumenmanschette (2) zum Umfassen eines Daumens (24) und
wenigstens einem daran befestigten Befestigungsband (12),
**dadurch gekennzeichnet, dass** das Befestigungsband (12) ein Loch (14) zum Hindurchstecken des Daumens (24) aufweist.

2. Daumenorthese (1) nach Anspruch 1, **dadurch gekennzeichnet, dass** die Daumenmanschette (2) wenigstens eine Schiene (20) zum Stabilisieren wenigstens eines Teils des Daumens (24) aufweist.

3. Daumenorthese (1) nach Anspruch 2, **dadurch gekennzeichnet, dass** die wenigstens eine Schiene (20) aus einem an eine Form des Daumens (24) anformbaren Material besteht.

4. Daumenorthese (1) nach Anspruch 3, **dadurch gekennzeichnet, dass** das anformbare Material ein Kunststoff oder ein Metall ist.

5. Daumenorthese (1) nach einem der Ansprüche 2 bis 4, **dadurch gekennzeichnet, dass** die Schiene (20) an einer Innenseite (6) der Daumenmanschette (2) angeordnet ist.

6. Daumenorthese (1) nach einem der Ansprüche 2 bis 5, **dadurch gekennzeichnet, dass** die Schiene (20) in die Daumenmanschette (2), insbesondere in eine an der Daumenmanschette angeordnete Tasche (8), herausnehmbar eingeführt ist.

7. Daumenorthese (1) nach einem der vorstehenden Ansprüche, **dadurch gekennzeichnet, dass** das Befestigungsband (12) zumindest abschnittsweise aus einem elastischen Material besteht.

8. Daumenorthese (1) nach einem der vorstehenden Ansprüche, **dadurch gekennzeichnet, dass** das Loch (14) in dem Befestigungsband (12) von unelastischem Material umgeben ist.

9. Daumenorthese (1) nach einem der vorstehenden Ansprüche, **dadurch gekennzeichnet, dass** das Befestigungsband (12) ein der Daumenmanschette (2) abgewandtes Ende (16) aufweist, das an der Daumenmanschette (2) befestigbar ist.

10. Daumenorthese (1) nach Anspruch 9, **dadurch gekennzeichnet, dass** an dem Ende (16) des Befestigungsbandes (12) und an der Daumenmanschette (2) Klettverschlusselemente (18) angeordnet sind.

## Claims

1. Thumb orthosis (1), comprising
a thumb cuff (2) for encompassing a thumb (24), and
at least one fastening strap (12) fastened thereto,
**characterized in that** the fastening strap (12) has a hole (14) through which the thumb (24) is passed.

2. Thumb orthosis (1) according to Claim 1, **characterized in that** the thumb cuff (2) has at least one splint (20) for stabilizing at least part of the thumb (24).

3. Thumb orthosis (1) according to Claim 2, **characterized in that** the at least one splint (20) is made of a material conformable to a shape of the thumb (24).

4. Thumb orthosis (1) according to Claim 3, **characterized in that** the conformable material is a plastic or a metal.

5. Thumb orthosis (1) according to one of Claims 2 to 4, **characterized in that** the splint (20) is arranged at an inner face (6) of the thumb cuff (2).

6. Thumb orthosis (1) according to one of Claims 2 to 5, **characterized in that** the splint (20) is inserted into the thumb cuff (2), in particular into a pocket (8) arranged at the thumb cuff, in such a way as to be removable therefrom.

7. Thumb orthosis (1) according to one of the preceding claims, **characterized in that** the fastening strap (12) is made at least in part from an elastic material.

8. Thumb orthosis (1) according to one of the preceding claims, **characterized in that** the hole (14) in the fastening strap (12) is surrounded by non-elastic material.

9. Thumb orthosis (1) according to one of the preceding claims, **characterized in that** the fastening strap (12) has an end (16) which is directed away from the thumb cuff (2) and which can be fastened to the thumb cuff (2).

10. Thumb orthosis (1) according to Claim 9, **characterized in that** hook-and-loop closure elements (18) are arranged at the end (16) of the fastening strap (12) and at the thumb cuff (2).

## Revendications

1. Orthèse de pouce (1) comportant
une manchette de pouce (2) pour entourer un pouce (24), et
au moins un ruban de fixation (12) fixé sur celle-ci,
**caractérisée en ce que** le ruban de fixation (12) présente un trou (14) pour faire traverser le pouce (24).

2. Orthèse de pouce (1) selon la revendication 1, **caractérisée en ce que** la manchette de pouce (2) présente au moins une attelle (20) pour stabiliser au moins une partie du pouce (24).

3. Orthèse de pouce (1) selon la revendication 2, **caractérisée en ce que** ladite au moins une attelle (20) est constituée en un matériau adaptable à une forme du pouce (24).

4. Orthèse de pouce (1) selon la revendication 3, **caractérisée en ce que** le matériau adaptable est une matière plastique ou un métal.

5. Orthèse de pouce (1) selon l'une des revendications 2 à 4, **caractérisée en ce que** l'attelle (20) est agencée sur un côté intérieur (6) de la manchette de pouce (2).

6. Orthèse de pouce (1) selon l'une des revendications 2 à 5, **caractérisée en ce que** l'attelle (20) est insérée de façon extractible dans la manchette de pouce (2), en particulier dans une poche (8) agencée dans la manchette de pouce.

7. Orthèse de pouce (1) selon l'une des revendications précédentes, **caractérisée en ce que** le ruban de fixation (12) est constitué au moins localement en un matériau élastique.

8. Orthèse de pouce (1) selon l'une des revendications précédentes, **caractérisée en ce que** le trou (14) dans le ruban de fixation (12) est entouré par un matériau inélastique.

9. Orthèse de pouce (1) selon l'une des revendications précédentes, **caractérisée en ce que** le ruban de fixation (12) présente une extrémité (16) détournée de la manchette de pouce (2), qui est susceptible d'être fixée sur la manchette de pouce (2).

10. Orthèse de pouce (1) selon la revendication 9, **caractérisée en ce que** des éléments de fermeture à bouclette et à crochets (18) sont agencés à l'extrémité (16) du ruban de fixation (12) et sur la manchette de pouce (2).
